# FASCICULE DE BREVET EUROPEEN

(11) **EP 2 689 026 B1**
(45) Date de publication et mention de la délivrance du brevet: **08.03.2017**
(21) Numéro de dépôt: 12714799.9
(22) Date de dépôt: 16.03.2012
(51) Int. Cl.: C12Q 1/04, C12N 1/20

(54) **DETECTION DE BACTERIES PRESENTANT UNE RESISTANCE AUX CARBAPENEMES**
NACHWEIS VON GEGEN CARBAPENEME RESISTENTEN BAKTERIEN
DETECTION OF BACTERIA HAVING A RESISTANCE TO CARBAPENEMS

(30) Priorité: 25.03.2011 FR 1152477
(43) Date de publication de la demande: 29.01.2014
(73) Titulaire: Biomérieux, 69280 Marcy l'Étoile (FR); Ghirardi, Sandrine, 69290 Saint Genis les Ollières (FR)
(72) Inventeur: GHIRARDI, Sandrine, F-69290 Saint Genis les Ollières (FR); PERRY, John, High Heaton Newcastle upon Tyne NE7 7BH (GB); ZAMBARDI, Gilles, F-38230 Tignieu Jameyzieu (FR)
(86) Numéro de dépôt international: PCT/FR2012/050556
(87) Numéro de publication internationale: WO 2012/131216

(56) Documents cités:
- WO-A1-2010/010083
- FR-A1- 2 925 070
- Z. SAMRA ET AL: "Evaluation of CHROMagar KPC for Rapid Detection of Carbapenem-Resistant Enterobacteriaceae", JOURNAL OF CLINICAL MICROBIOLOGY, vol. 46, no. 9, 16 juillet 2008 (2008-07-16), pages 3110-3111, XP055018354, ISSN: 0095-1137, DOI: 10.1128/JCM.00249-08
- THEOFANO PANAGEA ET AL: "Evaluation of CHROMagar(TM) KPC for the detection of carbapenemase-producing Enterobacteriaceae in rectal surveillance cultures", INTERNATIONAL JOURNAL OF ANTIMICROBIAL AGENTS, vol. 37, no. 2, 1 février 2011 (2011-02-01), pages 124-128, XP055017319, ISSN: 0924-8579, DOI: 10.1016/j.ijantimicag.2010.10.010
- S. MUSHTAQ ET AL: "Activity of faropenem against cephalosporin-resistant Enterobacteriaceae", JOURNAL OF ANTIMICROBIAL CHEMOTHERAPY, vol. 59, no. 5, 15 mars 2007 (2007-03-15), pages 1025-1030, XP055017372, ISSN: 0305-7453, DOI: 10.1093/jac/dkm063

## Description

La présente invention porte sur un procédé de détection et d'identification adapté au criblage de bactéries résistantes aux carbapénèmes.

L'augmentation de la résistance aux antibiotiques de type beta-lactames, tels les pénicillines et les céphalosporines, rend complexe le traitement des infections causées par des souches de bactéries Gram négatives. Ces antibiotiques sont alors remplacés par d'autres antimicrobiens à large spectre. Parmi ces antimicrobiens à large spectre, les carbapénèmes ont pris une place importante, en particulier pour traiter des patients hospitalisés. Les carbapénèmes sont actifs contre la majorité des bactéries aérobies Gram positives et Gram négatives ainsi que sur certaines bactéries anaérobies.

Cependant, de plus en plus de souches résistantes aux carbapénèmes apparaissent chez des patients hospitalisés.

Les bactéries concernées sont, de façon non exhaustive, *Escherichia coli, Enterobacter cloacae, Enterobacter aerogenes, Citrobacter sp., Klebsiella pneumoniae, Klebsiella oxytoca, Pseudomonas aeruginosa, Providencia rettgeri, Pseudomonas putida, Stenotrophomonas maltophilia, Acinetobacter baumanii, Comamonas sp., Aeromonas sp., Morganella morganii, Enterococcus sp., Proteus mirabilis, Salmonella senftenberg, Serratia marcescens, Salmonella typhimurium etc.*

La susceptibilité réduite aux carbapénèmes peut être due :
- à l'expression d'un gène de résistance aux beta-lactamines :
   (i) hyperproduction des beta-lactamases de type ampC et/ou
   (ii) ESBL (beta-lactamase à spectre étendu),
   combinée avec des modifications de la perméabilité de la paroi cellulaire (résistance par imperméabilité) et/ou avec l'efflux actif des antibiotiques (Pages et al., 2009 ; PloS ONE, 4 (3)); et/ou
- à l'existence d'enzymes dégradant les carbapénèmes, appelées carbapénémases.

Les gènes de carbapénémases sont susceptibles d'être présents dans les chromosomes et/ou dans des plasmides. Du fait de cette présence sous forme de plasmides, ces résistances de type enzymatiques sont susceptibles de se disséminer de façon très importante et présentent, en conséquence, un risque majeur en terme d'épidémiologie.

L'homme du métier a des difficultés pour détecter et/ou identifier aisément les souches de bactéries résistantes aux carbapénèmes.

Une méthode de caractérisation par utilisation d'un milieu chromogène comprenant du méropénème et/ou de l'ertapénème a été proposée dans la demande WO 2010/010083 et mise en oeuvre dans les milieux CHROMagar® KPC (Samra et al., 2008 ; J. Clin. Microbiol., 46 (9): 3110-3111; CHROMagar™, Paris, France) et COLOREX™ KPC (BioMed Diagnostics Inc). Ce milieu ne permet pas la détection de l'ensemble des souches productrices de carbapénémases, notamment les souches de type NDM-1, apparues récemment (Nordmann et al., 2011 ; J Clin Microbiol., 49(2) : 718-721). Dans le contexte épidémiologique actuel, et notamment avec l'émergence de ces nouvelles résistances NDM-1, il reste à améliorer, en sensibilité et/ou en spécificité, le criblage de l'ensemble des mécanismes de résistance aux carbapénèmes.

Le brevet FR 2 925 070 décrit un procédé de détection directe de bactéries productrices de beta-lactamase à spectre étendu (BLSE) et/ou de bactéries carbapénèmes résistantes.

Le faropénème et le doripénème sont des carbapénèmes développés et testés récemment (Mushtaq et al., 2007 ; Journal of Antimicrobial Chemotherapy, 59 : 1025-1030 - Lee et al., 2011, Microbial Drug Resistance). A la connaissance de la Demanderesse, ils n'ont jamais été utilisés pour détecter et/ou identifier, directement dans des échantillons biologiques, des souches bactériennes résistantes aux carbapénèmes.

La Demanderesse a montré qu'il est possible d'améliorer la détection des souches résistantes aux carbapénèmes, plus particulièrement l'ensemble des souches productrices de carbapénémases, incluant les carbapénémases de type KPC et les carbapénémases de type NDM. Ceci répond aux exigences liées au contexte épidémiologique actuel, en particulier avec l'émergence de nouvelles résistances telles que les NDM-1, et permet le criblage de l'ensemble des mécanismes de résistance liés à la production de tous types connus de carbapénémases.

A cet égard, la présente invention est relative à un procédé pour détecter et/ou identifier, dans un échantillon biologique, des bactéries présentant une résistance aux carbapénèmes, comprenant les étapes consistant à :
a) mettre en contact ledit échantillon avec un milieu réactionnel comprenant au moins un agent chromogène et du faropenem et/ou du doripenem ;
b) incuber l'ensemble de façon à permettre la croissance des bactéries ;
c) détecter les souches présentant une résistance aux carbapénèmes.

La Demanderesse a montré que l'addition de faropenem dans un milieu chromogène permet la croissance d'une majorité de bactéries productrices de carbapénémases, tout en inhibant une majorité de souches n'en produisant pas, telles des souches sauvages, des souches productrices de beta-lactamases à spectre étendu ou de céphalosporinases de haut niveau, par exemple. Les essais ont permis de démontrer une sensibilité et une spécificité supérieures à celles d'un milieu chromogène utilisant du méropénème.

Selon un premier mode de réalisation, la présente invention correspond à un procédé de détection et/ou d'identification, dans un échantillon biologique, de bactéries présentant une résistance aux carbapénèmes, comprenant les étapes consistant à :
a) mettre en contact ledit échantillon avec un milieu de culture comprenant au moins un substrat chromogène, et au moins du faropénème ;
b) incuber l'ensemble de façon à permettre la croissance des bactéries ;
c) détecter les souches présentant une résistance aux carbapénèmes.

Selon un mode préférentiel de réalisation de l'invention, les bactéries sont des bactéries de type NDM-1. Préférentiellement, les concentrations en faropénème sont comprises entre 2 et 32 mg/L

Avantageusement, le milieu mis en oeuvre à l'étape a) comprend également de la cloxacilline et/ou une combinaison de cloxacilline et de PAbetaN. Ces composés apportent un niveau de sélection supplémentaire et permettent de distinguer les résistances par imperméabilité et les autres résistances non enzymatiques des résistances par production de carbapénémase.

Par échantillon biologique, on entend une petite partie ou petite quantité isolée d'une entité pour l'analyse. Ce peut être un échantillon clinique, humain ou animal, issu d'un prélèvement de liquide biologique, ou un échantillon alimentaire, issu de tout type d'aliment, ou un échantillon de l'environnement de production ou de transformation d'aliments. Cet échantillon peut être ainsi liquide ou solide. On peut citer d'une manière non limitative, un échantillon clinique de sang total, de sérum, de plasma, d'urines, de fécès, de prélèvements de nez, de gorges, de peau, de plaies, de liquide céphalo-rachidien, un échantillon alimentaire d'eau, de boissons tels que le lait, un jus de fruits, de yaourt, de viande, d'oeufs, de légumes, de mayonnaise, de fromage, de poisson..., un échantillon alimentaire issu d'une alimentation destinée aux animaux, tel que notamment un échantillon issu de farines animales, un échantillon pour contrôle de surface ou d'eau. Ce prélèvement peut être utilisé tel quel ou, préalablement à l'analyse, subir une préparation de type enrichissement, dilution, extraction, concentration, purification, selon des méthodes connues de l'homme du métier.

Par milieu réactionnel, on entend un milieu comprenant tous les éléments nécessaires à l'expression d'un métabolisme et/ou à la croissance de microorganismes. Le milieu réactionnel peut être solide, semi-solide ou liquide. Par milieu solide, on entend par exemple un milieu gélifié. L'agar est l'agent gélifiant traditionnel en microbiologie pour la culture des microorganismes, mais il est possible d'utiliser de la gélatine, de l'agarose ou d'autres gélifiants naturels ou artificiels. Un certain nombre de préparations sont disponibles dans le commerce, comme par exemple l'agar Columbia, la gélose Trypcase-soja, la gélose Mac Conkey ou plus généralement celles décrites dans le Handbook of Microbiological Media (CRC Press).

Le milieu réactionnel peut comprendre un ou plusieurs éléments en combinaison, tels que des acides aminés, des peptones, des hydrates de carbone, des nucléotides, des minéraux, des vitamines ... Le milieu peut comprendre également un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant, un ou plusieurs indicateurs métaboliques, un ou plusieurs régulateurs métaboliques...

Le milieu réactionnel peut être un milieu de révélation ou un milieu de culture et de révélation. Dans le premier cas, la culture des microorganismes est effectuée avant ensemencement et, dans le deuxième cas, le milieu de détection et/ou d'identification constitue également le milieu de culture.

L'homme du métier peut également utiliser une bi-boite, permettant de comparer aisément deux milieux, comprenant différents substrats ou différents mélanges sélectifs, sur lesquels on aura déposé un même échantillon biologique.

Le milieu réactionnel peut comprendre un ou plusieurs agents sélectifs. Par agent sélectif, on entend tout composé susceptible d'empêcher ou de ralentir la croissance d'un microorganisme autre que le microorganisme cible. Sans être limitatif, une concentration comprise entre 0,01 mg/l et 5 g/l est particulièrement adaptée à la présente invention.

On peut citer comme agent sélectif, les antibiotiques, les antifongiques, les sels biliaires, le crystal violet, la fushine basique, le vert brillant, ... Par antibiotique, on entend tout composé susceptible d'empêcher ou de ralentir la croissance d'une bactérie. Ils appartiennent notamment aux groupes des beta-lactamines, des glycopeptides, des aminosides, des polypeptides, des sulfamides, des quinolones. A titre indicatif, on peut citer notamment les antibiotiques cloxacilline, cefotaxime, cefsulodine, ceftazidime, cefoxitine, ceftriaxone, cefpodoxime, aztréonam, vancomycine, gentamicine, Triméthoprime, tobramycine, moxalactam, fosfomycine, D-cylcosérine, Polymixine, Colistine, les quinolones telles que l'acide nalidixique. Par antifongique, on entend tout composé susceptible d'empêcher ou de ralentir la croissance d'une levure ou d'une moisissure. A titre indicatif, on peut citer notamment l'amphotéricine B, le fluconazole, l'itraconazole, le voriconazole, la cycloheximide.

La cloxacilline correspond à un antibiotique de la classe des pénicillines. Elle est utilisée *in vitro* pour inhiber certaines beta-lactamases (Giske et al., 2010 ; Clin. Microbiol. Infect. ; 17 (4) : 552-6). La dicloxacilline et la flucloxacilline sont avantageusement considérées comme équivalentes à la cloxacilline. De façon préférentielle, la cloxacilline est utilisée à une concentration comprise entre 25 et 300 mg/l.

Le PABN ou PAbetaN correspond au phenylalanine-arginine-beta-naphtylamide. Ce composé est connu comme inhibiteur des pompes à efflux, permettant par exemple de diminuer la concentration minimale inhibitrice (CMI) du chloramphenicol vis-à-vis des souches *d'Enterobacter aerogenes* (Mallea et al., 2002 ; Biochemical and Biophysical Research Communications 293 : 1370-3). De façon préférentielle, le PAbetaN est utilisé à une concentration comprise ente 1 et 50 mg/l.

Par substrat chromogène, on entend un substrat permettant la détection d'une activité enzymatique ou métabolique des microorganismes cibles grâce à un signal détectable directement ou indirectement. Pour une détection directe, ce substrat peut être lié à une partie faisant office de marqueur, fluorescent ou coloré (Orenga et al., 2009 ; J. Microbiol. Methods ; 79(2):139-55). Pour une détection indirecte, le milieu réactionnel selon l'invention peut comporter en sus un indicateur de pH, sensible à la variation de pH induite par la consommation du substrat et révélant le métabolisme des microorganismes cibles. Ledit indicateur de pH peut être un chromophore ou un fluorophore. On citera comme exemples de chromophores le bromocresol pourpre, le bleu de bromothymol, le rouge neutre, le bleu d'aniline, le bleu de bromocresol. Les fluorophores comprennent par exemple la 4-méthylumbelliferone, les dérivés de l'hydroxycoumarine ou les dérivés de la résorufine.

Selon la présente invention, le substrat chromogène est préférentiellement choisi parmi les substrats à base d'Indoxyl (3-Indoxyl, 5-Bromo-3-indoxyl, 5-lodo-3-indoxyl, 4-Chloro-3-indoxyl, 5-Bromo-4-chloro-3-indoxyl, 5-Bromo-6-chloro-3-indoxyl, 6-Bromo-3-indoxyl, 6-Chloro-3-indoxyl, 6-Fluoro-3-indoxyl, 5-Bromo-4-chloro-N-méthyl-3-indoxyl, N-Méthyl-3-indoxyl, Aldol™ ...); d'umbelliférone (4-Méthylumbelliférone, Cyclohexenoesculétine, ...); d'Alizarine; de p-Naphtolbenzéine; de Nitrophénol (ortho-Nitrophénol, para-Nitrophénol, ...); d'Hydroxyquinoline; de Cathécol (Cathécol, Dihydroxyflavone, Hydroxyflavone, ...); de Résorufine; de Chlorophénol Red; de Fluorescéine; d'Aminophénol (para-Aminophénol, Dichloro-aminophénol, ...); de Naphtol (alpha-Naphtol, 2-Naphtol, Naphtol-ASBI, ...) ; d'Aminocoumarine (7-Amino-4-méthyl-coumarine, ...); de Naphtylamide; d'Acridine (Amino-phényl-acridine...); d'Amino-phénoxazine (Amino-benzophénoxazinone, Amino-pentyl-resorufine, ...).

A titre indicatif, les activités enzymatiques ciblées pas les substrats chromogènes peuvent appartenir au groupe des hydrolases, préférentiellement aux groupes des osidases, des estérases ou des peptidases.

A titre indicatif, les substrats utilisés pour la détection d'une activité beta-glucuronidase peuvent notamment être le 4-Méthylumbelliféryl-beta-glucuronide, le 5-Bromo-4-chloro-3-indolyl-beta-glucuronide, le 5-Bromo-6-chloro-3-indolyl-beta-glucuronide, le 6-Chloro-3-indolyl-beta-glucuronide, l'Alizarine-beta-glucuronide, le Cyclohexenoesculetine-beta-glucuronide ou leurs sels.

Les substrats utilisés pour la détection d'une activité beta-galactosidase peuvent notamment être le 4-Méthylumbelliféryl-beta-galactoside, le 5-Bromo-4-chloro-3-indolyl-beta-galactoside, le 5-Bromo-6-chloro-3-indolyl-beta-galactoside, le 6-Chloro-3-indolyl-beta-galactoside, l'Alizarine-beta-galactoside, le Cyclohexenoesculetine-beta-galactoside ou leurs sels.

Les substrats utilisés pour la détection d'une activité beta-glucosidase peuvent notamment être le 4-Méthylumbelliféryl-beta-glucoside, le 5-Bromo-4-chloro-3-indolyl-beta-glucoside, le 5-Bromo-4-chloro-3-indolyl-N-méthyl-beta-glucoside, le 5-Bromo-6-chloro-3-indolyl-beta-glucoside, le 6-Chloro-3-indolyl-beta-glucoside, l'Alizarine-beta-glucoside, le Cyclohexenoesculetine-beta-glucoside, le Nitrophényl-beta-glucoside, le Dichloroaminophényl-glucoside ou leurs sels.

A titre indicatif, les substrats utilisés pour la détection d'une activité estérase peuvent notamment être les esters d'acides gras linéaires saturés ou insaturés, ayant entre 6 et 14 carbones, préférentiellement entre 7 et 9 carbones et de 4-Méthylumbelliférone, 5-Bromo-4-chloro-3-indoxyl, 5-Bromo-6-chloro-3-indoxyl, 6-Chloro-3-indoxyl, 5-Bromo-3-indolyl ou d'Alizarine ou leurs sels. Préférentiellement, ils sont choisis parmi 4-Méthylumbellifyl-octanoate, 5-Bromo-4-chloro-3-indoxyl-octanoate, 5-Bromo-6-chloro-3-indoxyl-octanoate, 6-Chloro-3-indoxyl-octanoate, 5-Bromo-3-indolyl-octanoate ou d'Alizarine-octanoate.

Les substrats utilisés pour la détection d'une activité désaminase peuvent notamment être le L-Tryptophane, la L-Phénylalanine, la L-Tyrosine et la L-Histidine. Les substrats utilisés pour la détection d'une activité sulfatase peuvent notamment être le 4-Méthylumbelliféryl-sulfate, le 5-Bromo-4-chloro-3-indoxyl-sulfate, le 5-Bromo-6-chloro-3-indoxyl-sulfate, le 3-Indoxyl-sulfate, le Phenolphtaléine-disulfate ou leurs sels.

Préférentiellement, le substrat chromogène est choisi parmi : le 5-Bromo-4-chloro-3-indoxyl-beta-D-glucopyranoside (X-glucoside), le 5-Bromo-6-chloro-3-indoxyl-beta-D-galactopyranoside (Magenta beta-Gal), le 6-Chloro-3-indoxyl-beta-D-glucuronide (Rose beta Gur), le 5-Bromo-4-chloro-3-indoxyl-N-méthyl-beta-D-glucopyranoside (Green A beta Glu), le Méthyl-beta-D-glucopyranoside (methyl beta D glucoside), le L-Tryptophane..

Par incuber, on entend porter et maintenir entre 1 et 48 heures, préférentiellement entre 4 et 24 heures, plus préférentiellement entre 16 et 24 heures, à une température appropriée, généralement comprise entre 20 et 50°C, préférentiellement entre 30 et 40 °C.

Par détecter, on entend déceler à l'oeil nu ou à l'aide d'un appareil optique l'existence d'une croissance des bactéries cibles. Avantageusement, lorsque le milieu mis en oeuvre comprend un substrat chromogène, la détection peut également permettre une identification des bactéries cibles. La détection se fait à l'aide d'un appareil optique pour les substrats fluorescents, ou à l'oeil nu ou à l'aide d'un appareil optique pour les substrats colorés.

Par résistance enzymatique aux carbapénèmes, on entend, tel qu'indiqué *supra,* la résistance aux antibiotiques de type carbapénèmes due à l'expression, par les bactéries cibles, de carbapénémases.

Les bactéries résistantes aux carbapénèmes les plus fréquemment rencontrées sont, tel qu'indiqué *supra : Escherichia coli, Enterobacter cloacae, Enterobacter aerogenes, Citrobacter sp., Klebsiella pneumoniae, Klebsiella oxytoca, Pseudomonas aeruginosa, Providencia rettgeri, Pseudomonas putida, Stenotrophomonas maltophilia, Acinetobacter baumanii, Comamonas sp., Aeromonas sp., Morganella morganii, Enterococcus sp., Proteus mirabilis, Salmonella senftenberg, Serratia marcescens, Salmonella typhimurium* etc.

La présente invention porte également sur un milieu de culture pour détecter et/ou identifier des bactéries présentant une résistance aux carbapénèmes, ledit milieu de culture correspondant à un milieu de culture de base, comprenant en outre au moins un substrat chromogène, et au moins du faropénème.

Les exemples développés ci-dessous ont pour but de faciliter la compréhension de l'invention. Ils sont donnés à titre explicatif et ne sauraient limiter la portée de l'invention.

### EXEMPLES

### Exemple 1

Essai du faropénème sur souches pures.

### 1. Milieux et microorganismes

Soixante dix neuf souches de bactéries Gram négatives, dont 69 entérobactéries et 10 non entérobactéries (bactéries non fermentants), dont les caractéristiques de résistances sont détaillées dans le Tableau I ont été testées sur des milieux contenant différentes concentrations de faropénème afin d'établir la sensibilité/spécificité de chaque formulation. La lecture des boîtes a été effectuée après 24h d'incubation à 37°C.

**Tableau I : mécanismes de résistance caractérisant les souches testées**

| | Nombre de souches |
|---|---|
| BLSE ou céphalosporinase (BLSE ou Case) | 15 |
| Carbapénémase de Classe A type KPC (KPC) | 34 |
| Carbapénémase de Classe A non KPC (Carba A) | 8 |
| Carbapénémase de Classe B (Carba B) | 10 |
| Carbapénémase de Classe D (Oxa) | 6 |
| Résistance par imperméabilité (RI) | 6 |

Le milieu utilisé est un milieu gélosé classique comprenant au moins un substrat chromogène, et supplémenté en faropénème aux concentrations suivantes :

| | Milieu 1 | Milieu 2 | Milieu 3 | Milieu 4 | Milieu 5 | Milieu 6 | Milieu 7 | Milieu 8 |
|---|---|---|---|---|---|---|---|---|
| Faropénème (mg/L) | 0 | 0,5 | 1 | 2 | 4 | 8 | 16 | 32 |

### 2. Essai

Les milieux sont répartis en boîtes carrées 120X120.

L'ensemencement est effectué à partir de pré-cultures de 24h à 37°C sur gélose trypcase soja.

Pour chaque souche, une suspension à 0,5 McF en eau physiologique est réalisée puis diluée 1/100.

Chaque suspension est ensemencée en spot (1 à 2 µL) sur chaque milieu à l'aide d'un inoculateur multi-points selon la méthode de dilution en gélose (DEG).

Des lectures sont effectuées après 24 heures d'incubation à 37°C.

Pour l'interprétation, les cas suivants sont considérés comme correspondant à une inhibition de croissance :
o absence de croissance bactérienne
o nombre de colonies ≤ 3

La présence de 4 colonies ou plus est considérée comme croissance positive.

### 3. Résultats et interprétation

Sensibilité et spécificité de détection sont indiqués dans le Tableau II.

**Tableau II : sensibilité/spécificité de détection des souches productrices de carbapénémases sur un milieu contenant 16 mg/L de faropénème.**

| | Inoculum : 0,5 McF | Inoculum : 0,5 McF dilué 1/100 |
|---|---|---|
| Sensibilité de détection | 53/58 | 53/58 |
| | 91,4% | 91,4% |
| Spécificité de détection | 14/21 | 15/21 |
| | 66,7% | 71,4% |

Le faropénème, utilisé par exemple à 16 mg/L, permet une très bonne discrimination entre les bactéries productrices de carbapénémases et les autres types de résistances.

La CMI de la totalité des souches KPC est toujours >32, quel que soit l'inoculum testé. Toutes les souches KPC se développent sur le milieu comprenant du faropénème à 16 mg/L.

### Exemple 2

### Essai du doripénème sur souches pures

### 1. Milieux et microorganismes

Cent souches de bactéries Gram négatives, dont 98 entérobactéries et 2 non entérobactéries (bactéries non fermentants), dont les caractéristiques de résistances sont détaillées dans le Tableau III ont été testées sur des milieux contenant différentes concentrations de doripénème afin d'établir la sensibilité et la spécificité de chaque formulation. Un milieu contenant du méropénème à 0,125 mg/L a été également testé. Le milieu chromogénique UriSelect® (Biorad) est utilisé comme témoin de croissance. La lecture des boîtes a été effectuée après 24h et 48h d'incubation à 37°C.

**Tableau III : mécanismes de résistance caractérisant les souches testées**

| | Nombre de souches |
|---|---|
| BLSE | 42 |
| Céphalosporinase : AmpC (30), autres (4) | 34 |
| Carbapénémase de Classe A type KPC (KPC) | 5 |
| Carbapénémase de Classe B (Carba B) | 19 |

Le milieu utilisé est un milieu gélosé classique comprenant au moins un substrat chromogène, et supplémenté en carbapénème aux concentrations suivantes :

| | Milieu A | Milieu B | Milieu C | Milieu D | Milieu E |
|---|---|---|---|---|---|
| Doripénème (mg/L) | 0 | 0,065 | 0,125 | 0,25 | 0 |
| Méropénème (mg/L) | 0 | 0 | 0 | 0 | 0,125 |

### 2. Essai

Les milieux sont répartis en boîtes 90 mm.

Chaque souche est ensemencée en spot sur chaque milieu, à l'aide de 1 µL (contenant environ 10⁴ microorganismes) d'une suspension de 0,5 McF diluée.

Des lectures sont effectuées après 24 et 48 heures d'incubation à 37°C.

### 3. Résultats et interprétation

Les résultats sont regroupés dans le Tableau IV.

**Tableau IV : pourcentage de souches de chaque catégorie de résistance se développant sur les différents milieux.**

| | Uriselect® (Biorad) | Milieu A | Milieu B 0,065 mg/L doripénème | Milieu C 0,125 mg/L doripénème | Milieu D 0,25 mg/L doripénème | Milieu E 0,125 mg/L méropénème |
|---|---|---|---|---|---|---|
| Carbapénémases | 100 | 100 | 83 | 50 | 33 | 50 |
| Autres | 100 | 98,7 | 8 | 5 | 4 | 5 |

### Exemple 3

Comparaison du milieu comprenant du doripénème avec un milieu commercialisé

### 1. Milieux et microorganismes

Cent souches d'entérobactéries dont 25 productrices de carbapénémases et dont les caractéristiques de résistances sont détaillées dans le Tableau V ont été testées sur le milieu B décrit *supra* (0,065 mg/L de doripénème) et sur le milieu COLOREX™ KPC. Ce dernier milieu est fourni par BioMed Diagnostics Inc et correspond au milieu prêt à l'emploi CHROMagar™ KPC.

**Tableau V : mécanismes de résistance caractérisant les souches testées**

| | Nombre de souches |
|---|---|
| BLSE | 42 |
| Céphalosporinase AmpC (29), autres (4) | 33 |
| Carbapénémase de Classe A type KPC | 6 |
| Carbapénémase de Classe B (Carba B) | 19 |

### 2. Essai

Chaque souche est ensemencée en spot sur chaque milieu, à l'aide de 1 µL (contenant environ 10⁴ microorganismes) d'une suspension de 0,5 McF diluée.

Des lectures sont effectuées après 24 et 48 heures d'incubation à 37°C.

### 3. Résultats et interprétation

**Tableau VI : pourcentage de souches se développant sur les milieux**

| | COLOREX KPC 24 h | COLOREX KPC 48 h | Milieu B (0,065 mg/L doripénème) 24 h | Milieu B (0,065 mg/L doripénème) 48 h |
|---|---|---|---|---|
| % souches productrices de carbapénémases | 80 | 80 | 88 | 92 |
| % souches autres | 5 | 5 | 10,7 | 10,7 |

Le milieu contenant 0,065 mg/L de doripénème permet la croissance de 92 % des Entérobactéries productrices de carbapénémases testées, contre seulement 80% sur le milieu commercial, quel que soit le temps d'incubation. Le milieu comprenant du doripénème est donc supérieur au milieu commercial.

### Exemple 4

### Essais sur prélèvements cliniques

### 1. Milieux et prélèvements

Deux cents écouvillons (avec milieu de transport) chargés de populations bactériennes isolées de selles ont été ensemencés sur le milieu B indiqué *supra* (0,065 mg/L de doripénème), sur un milieu proche du milieu 7 indiqué *supra* (16 mg/L de faropénème) et sur le milieu COLOREX™ KPC.

### 2. Essai

0,5 mL d'eau stérile sont ajoutés aux tubes contenant écouvillon et milieu de transport. Les tubes sont vortexés.

10 µl de la suspension obtenue sont ensemencés en 4 quadrants sur chaque milieu. Des lectures des milieux sont effectuées après 24 heures d'incubation à 37°C.

### 3. Résultats et interprétation

**Tableau VII : résultats de criblage de souches productrices de carbapénémases à partir de populations bactériennes isolées de selles.**

| | Tous milieux confondus | Milieu B (0,065 mg/L doripénème) | KPC Colorex | milieu 7 (16 mg/L faropénème) |
|---|---|---|---|---|
| Aucune croissance | | 141 | 156 | 145 |
| Prélèvements Positifs Metallo-carbapénémase | | | | |
| *E. coli* | 30 | 30 | 12 | 30 |
| *E. cloacae* | 22 | 22 | 22 | 17 |
| *Citrobacter sp.* | 7 | 3 | 2 | 6 |
| *K. pneumoniae* | 3 | 0 | 2 | 2 |
| P. *rettgeri* | 2 | 0 | 2 | 0 |
| Total EPC¹: | 64 | 55 | 40 | 55 |
| Sensibilité (%) | | 85,9 | 62,5 | 85,9 |
| S. *maltophilia* | 4 | 4 | 3 | 4 |
| *A. baumannii* | 3 | 2 | 3 | 0 |
| *Ps putida* | 1 | 0 | 1 | 0 |
| *Comamonas aquatica* | 1 | 0 | 1 | 0 |
| *Aeromonas sp.* | 1 | 0 | 1 | 0 |
| Prélèvements Négatifs Metallo-carbapénémase | | | | |
| *E. coli* | 13 | 13 | 2 | 9 |
| *A. baumannii* | 4 | 3 | 3 | 1 |
| *P. aeruginosa* | 1 | 1 | 1 | 0 |
| *M. morganii* | 5 | 5 | 1 | 1 |
| Total faux positifs: | 33 | 28 | 16 | 15 |
| (Gram negatifs uniquement) | | | | |
| Enterococcus sp. | 13 | 11 | 0 | 13 |

| | | | | |
|---|---|---|---|---|
| ¹ Entérobactéries Productrices de Carbapénémases | | | | |

Les 64 souches Entérobactéries Productrices de Carbapénémases (EPC), de type NDM-1, ont été isolées de 37 prélèvements de selles Le milieu 7 comprenant 16 mg/L de faropénème présente les meilleures performances en terme de sensibilité (86%) et de spécificité (15 faux positifs Gram-). Le milieu B comprenant 0,065 mg/L de doripénème présente une sensibilité aussi élevée, mais une spécificité un peu moindre (28 faux positifs Gram-). Le milieu COLOREX™ KPC permet la détection de seulement 62,5% des EPC, pour une spécificité/sélectivité équivalente à celle du milieu comprenant du faropénème.

En conclusion, la méthode selon l'invention, de détection de bactéries résistantes aux carbapénèmes, démontre, à travers les exemples 1, 2, 3 et 4, l'amélioration qu'elle apporte à l'état de la technique, et ce, à la fois sur des souches de laboratoires et sur des prélèvements cliniques.

### Exemple 5

Test de stabilité des différents carbapénèmes en milieu gélosé.

Des milieux gélosés sur le modèle de ceux indiqués *supra* ont été préparés et conservés à une température comprise entre 2 et 8°C jusqu'à 9 semaines (63 jours). Des souches ESBL, AmpC, carbapénémases de classe B, RI ou KPC ont été ensemencées sur ces différents milieux à JO, J14, J42 et J63.

Ces tests ont permis de montrer que le doripénème et le faropénème présentent une stabilité en milieu gélosé proche de celle de l'ertapénème, et supérieure à celle de l'imipénème et du méropénème.

### Exemple 6

Impact de la cloxacilline et/ou du PAbetaN sur les Concentrations Minimales Inhibitrices (CMI) des souches résistantes par imperméabilité (RI) ou présentant un autre mécanisme de résistance non enzymatique, ou productrices de carbapénémases, en présence de faropenem.

### 1. Milieux et microorganismes

Soixante dix-sept souches de bactéries Gram négatives, dont 71 entérobactéries et 6 non entérobactéries (bactéries non fermentant), dont les caractéristiques de résistances sont détaillées dans le Tableau VIII, ont été testées sur des milieux contenant différentes concentrations de faropénème et de cloxacilline, avec ou sans PAbetaN, afin d'établir la sensibilité et la spécificité de chaque formulation. Les milieux testés sont décrits dans le Tableau IX.

**Tableau VIII : mécanismes de résistance aux antibiotiques caractérisant les souches testées.**

| | Nombre de souches |
|---|---|
| BLSE (7), Céphalosporinase AmpC (9) | 16 |
| Carbapénémase de Classe A type KPC (KPC) | 10 |
| Carbapénémase de Classe A autre que KPC (Class A) | 5 |
| Carbapénémase de Classe B type NDM-1 (NDM) | 15 |
| Carbapénémase de Classe B autre que NDM (Class B) | 6 |
| Carbapénémase de Classe D type OXA (OXA) | 6 |
| Résistance par imperméabilité (RI) | 19 |

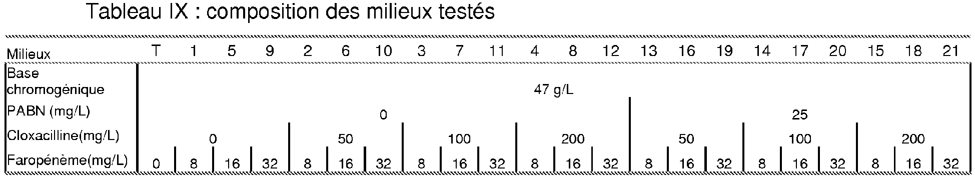

### 2. Essai

Les milieux chromogéniques sont répartis en boîtes carrées 120x120.

L'ensemencement est effectué à partir de pré-cultures de 24h à 37°C sur gélose trypcase soja. Pour chaque souche, une suspension à 0,5 McF en eau physiologique est réalisée. Chaque suspension est ensemencée en spot (1 à 2 µL) sur chaque milieu à l'aide d'un inoculateur multi-points selon la méthode de dilution en gélose (DEG). Des lectures sont effectuées après 24 heures d'incubation à 37°C.

### 3. Résultats

Les résultats sont regroupés dans le Tableau X.

Une absence de croissance bactérienne ou un nombre de colonies inférieur ou égal à 3 sont considérés comme correspondant à une inhibition de croissance. La présence de 4 colonies ou plus est considérée comme croissance positive.

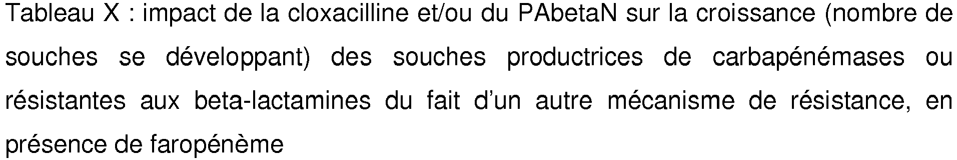

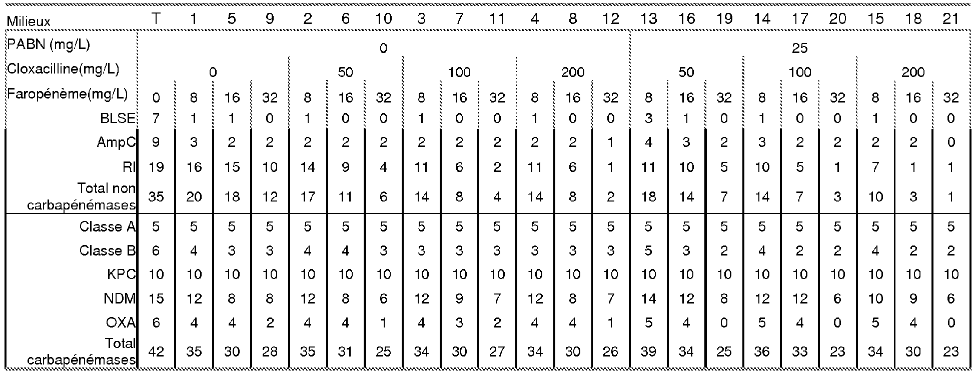

### 4. Interprétation

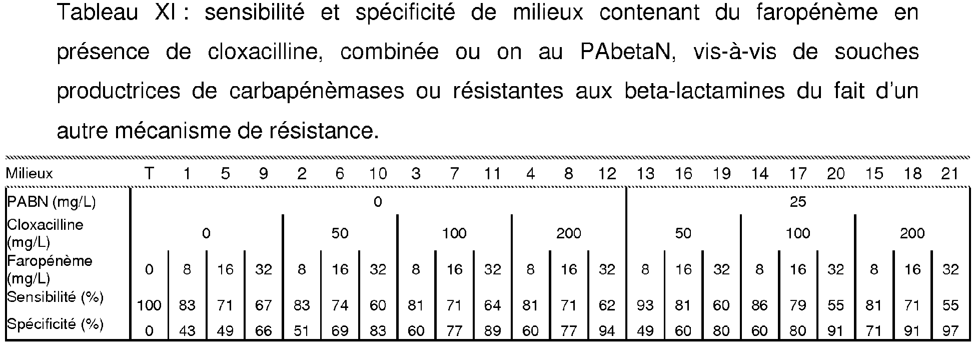

### 4a. Impact de la cloxacilline et du PAbetaN sur les souches RI

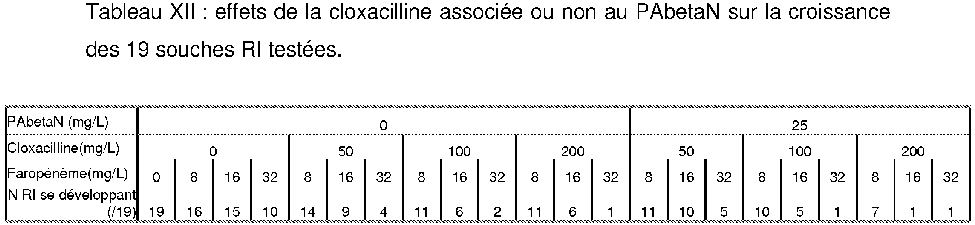

Le faropénème seul a peu d'impact sur ces souches, jusqu'à une concentration de 16 mg/L.

On observe une inhibition des souches RI dès ajout de 50 mg/L de cloxacilline, encore plus marquée pour les concentrations les plus élevées en faropénème testées (16 et 32 mg/L). Cet effet inhibiteur augmente avec la concentration de cloxacilline (par exemple, pour une concentration de 16 mg/L de faropénème, l'ajout de cloxacilline à 50 mg/L ou 200 mg/L permet respectivement l'inhibition de 6 et 9 souches de RI supplémentaires). On observe un effet combiné de la concentration en faropénème et de celle en cloxacilline sur les souches RI.

Ces résultats confirment également la meilleure inhibition des souches RI en présence de cloxacilline + PAbetaN. Cet effet est visible pour la plupart des concentrations de cloxacilline et faropénème testées, mais est très nettement marqué en présence de 200 mg/L de cloxacilline (quelle que soit la concentration en faropénème).

### 4b. Impact de la cloxacilline et du PAbetaN sur les souches productrices de carbapénémases

Les résultats obtenus en présence de faropénème confirment que la cloxacilline seule a peu ou pas d'impact sur la croissance des souches productrices de carbapénémases (aux 3 concentrations de faropénème testées). Ils confirment également que le fait d'associer le PAbetaN à la cloxacilline permet d'augmenter la CMI de certaines souches aux carbapénèmes tant que la concentration en faropénème reste inférieure à 32 mg/L. Ainsi, la sensibilité de détection en présence de 8 ou 16 mg/L de faropénème associé à 50 ou 100 mg/L de cloxacilline est supérieure en présence de PAbetaN. Pour 200 mg/L de cloxacilline et 8 ou 16 mg/L de faropénème, l'ajout de PAbetaN ne modifie pas la sensibilité de détection.

### 5. Conclusion

Ces résultats confirment le rôle inhibiteur de la cloxacilline sur les souches résistantes par imperméabilité, en présence d'un carbapénème. Ces résultats confirment également la meilleure inhibition des souches RI lorsque la cloxacilline est associée au PAbetaN. L'ajout de cloxacilline et de PAbetaN permet ainsi d'augmenter très fortement la spécificité du milieu.

L'ajout dans le milieu de cloxacilline (aux 3 concentrations testées) et de PAbetaN (25 mg/L) n'altère pas la détection des souches productrices de carbapénémases en présence de 8 ou 16 mg/L de faropénème. Il est confirmé que l'ajout de PAbetaN tend à améliorer leur détection à des concentrations de faropénème égales à 8 ou 16 mg/L, associées à la cloxacilline à 50 ou 100 mg/L.

En présence de faropénème, on confirme donc les résultats observés avec l'ertapénème (E-test), à savoir une meilleure inhibition des souches RI par l'ajout de cloxacilline et une amélioration de la détection des souches productrices de carbapénémases en présence de PAbetaN.

## Revendications

1. Procédé de détection de bactéries résistantes aux carbapénèmes dans un échantillon biologique, comprenant les étapes consistant à :
o mettre en contact ledit échantillon avec un milieu de culture comprenant au moins un substrat chromogène et au moins du faropénème,
o incuber l'ensemble de façon à permettre la croissance des bactéries, et
o détecter les souches correspondant aux bactéries résistantes aux carbapénèmes.

2. Procédé selon la revendication 1, dans lequel les bactéries sont des bactéries de type NDM-1.

3. Procédé selon l'une des revendications 1 à 2, dans lequel la concentration en faropénème est comprise entre 2 et 32 mg/L.

4. Procédé selon l'une des revendications 1 à 3, dans lequel le substrat chromogène détecte une activité choisie parmi : la glucuronidase, la glucosidase, la galactosidase, l'estérase, la sulfatase et la désaminase.

5. Procédé selon l'une des revendications 1 à 4, dans lequel le substrat chromogène est choisi parmi : le 5-Bromo-4-chloro-3-indoxyl-beta-D-glucopyranoside (X-glucoside), le 5-Bromo-6-chloro-3-indoxyl-beta-D-galactopyranoside (Magenta beta-Gal), le 6-Chloro-3-indoxyl-beta-D-glucuronide (Rose beta Gur), le 5-Bromo-4-chloro-3-indoxyl-N-méthyl-beta-D-glucopyranoside (Green A beta Glu), le L-Tryptophane

6. Procédé selon l'une des revendications 1 à 5, dans lequel le milieu réactionnel est un milieu de culture liquide ou solide.

7. Milieu de culture pour détecter et/ou identifier des bactéries présentant une résistance aux carbapénèmes, comprenant un milieu de culture de base, au moins un substrat chromogène, et au moins du faropénème.

## Patentansprüche

1. Verfahren für den Nachweis von carbapenemresistenten Bakterien in einer biologischen Probe, umfassend die aus Folgendem bestehenden Schritte:
∘ Inkontaktbringen der Probe mit einem Kulturmedium, das mindestens ein chromogenes Substrat und mindestens Faropenem umfasst,
∘ Inkubieren des Ganzen so, dass das Wachstum der Bakterien ermöglicht wird, und
∘ Nachweisen der Stämme, die den carbapenemresistenten Bakterien entsprechen.

2. Verfahren nach Anspruch 1, wobei es sich bei den Bakterien um Bakterien des Typs NDM-1 handelt.

3. Verfahren nach einem der Ansprüche 1 bis 2, wobei die Faropenemkonzentration zwischen 2 und 32 mg/l liegt.

4. Verfahren nach einem der Ansprüche 1 bis 3, wobei das chromogene Substrat eine Aktivität, ausgewählt aus Folgendem, nachweist: Glucuronidase, Glucosidase, Galactosidase, Esterase, Sulfatase und Desaminase.

5. Verfahren nach einem der Ansprüche 1 bis 4, wobei das chromogene Substrat aus Folgendem ausgewählt ist: 5-Brom-9-chlor-3-indoxyl-beta-D-glucopyranosid (X-Glucosid), 5-Brom-6-chlor-3-indoxyl-beta-D-galactopyranosid (Magenta-beta-Gal), 6-Chlor-3-indoxyl-beta-D-glucuronid (Rose-beta-Gur), 5-Brom-4-chlor-3-indoxyl-N-methyl-beta-D-glucopyranosid (Green-A-beta-Glu) und L-Tryptophan.

6. Verfahren nach einem der Ansprüche 1 bis 5, wobei es sich bei dem Reaktionsmedium um ein flüssiges oder festes Kulturmedium handelt.

7. Kulturmedium zum Nachweisen und/oder Identifizieren von Bakterien mit Carbapenemresistenz, umfassend ein Basalkulturmedium, mindestens ein chromogenes Substrat und mindestens Faropenem.

## Claims

1. A process for detecting bacteria which are resistant to carbapenems in a biological sample comprising the steps consisting in:
o contacting said sample with a culture medium comprising at least one chromogenic substrate and at least faropenem,
o incubating the whole so as to allow the bacteria to grow, and
o detecting the strains corresponding to the bacteria which are resistant to carbapenems.

2. The process according to Claim 1, wherein the bacteria are NDM-1 bacteria.

3. The process according to one of claims 1 to 2, wherein the faropenem concentration is between 2 and 32 mg/L.

4. The process according to one of claims 1 to 3, wherein the chromogenic substrate detects an activity chosen from: glucuronidase, glucosidase, galactosidase, esterase, sulfatase and deaminase.

5. The process according to one of claims 1 to 4, wherein the chromogenic substrate is chosen from: 5-Bromo-4-chloro-3-indoxyl-beta-D-glucopyranoside (X-glucoside), 5-Bromo-6-chloro-3-indoxyl-beta-D-galactopyranoside (Magenta beta-Gal), 6-Chloro-3-indoxyl-beta-D-glucuronide (Rose beta Gur), 5-Bromo-4-chloro-3-indoxyl-N-methyl-beta-D-glucopyranoside (Green A beta Glu), and L-Tryptophan.

6. The process according to one of claims 1 to 5, wherein the reaction medium is a liquid or solid culture medium.

7. A culture medium for detecting and/or identifying bacteria exhibiting a resistance to carbapenems, comprising a basic culture medium, at least one chromogenic substrate and at least faropenem.
